(19) Europäisches Patentamt
European Patent Office
Office européen des brevets

(11) **EP 4 074 823 B1**

(12) **EUROPEAN PATENT SPECIFICATION**

(45) Date of publication and mention of the grant of the patent:
**25.12.2024 Bulletin 2024/52**

(21) Application number: **20898809.7**

(22) Date of filing: **07.12.2020**

(51) International Patent Classification (IPC):
*C12N 15/09* (2006.01)    *C12Q 1/42* (2006.01)
*C12Q 1/6839* (2018.01)    *C12Q 1/6844* (2018.01)

(52) Cooperative Patent Classification (CPC):
(C-Sets available)
**C12Q 1/6844; C12Q 1/6846**    (Cont.)

(86) International application number:
**PCT/JP2020/045438**

(87) International publication number:
**WO 2021/117667 (17.06.2021 Gazette 2021/24)**

(54) **DETECTION METHOD**

NACHWEISVERFAHREN

PROCÉDÉ DE DÉTECTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.12.2019 JP 2019222153**

(43) Date of publication of application:
**19.10.2022 Bulletin 2022/42**

(73) Proprietor: **TOPPAN INC.**
**Tokyo 110-0016 (JP)**

(72) Inventors:
• **MAKINO, Yoichi**
  **Tokyo 110-0016 (JP)**
• **HORIUCHI, Yosuke**
  **Tokyo 110-0016 (JP)**

(74) Representative: **Plasseraud IP**
**104 Rue de Richelieu**
**CS92104**
**75080 Paris Cedex 02 (FR)**

(56) References cited:
WO-A1-2004/090517    JP-A- 2007 089 446
JP-A- 2016 527 918    JP-B2- 5 597 939
JP-B2- 6 183 471    US-A1- 2014 274 786

Remarks:
The complete document including Reference Table(s) and the Sequence Listing(s) can be downloaded from the EPO website

(52) Cooperative Patent Classification (CPC): (Cont.)

C-Sets
**C12Q 1/6844, C12Q 2563/103, C12Q 2563/159,
C12Q 2565/102;
C12Q 1/6846, C12Q 2563/103, C12Q 2563/159,
C12Q 2565/102**

**Description**

[Technical Field]

The present invention relates to detection methods.
The present application is based on and claims the benefit of priority from Japanese Patent Application No. 2019-222153 filed in Japan on December 9, 2019, the contents of which are incorporated herein by reference.

[Background Art]

By quantitatively detecting target molecules in a biological sample, early detection of disease and prediction of the effect of the medication can be performed. Nucleic acids such as DNA and RNA are quantified using real-time polymerase chain reaction (PCR) techniques or the like.
Recent years have seen an increasing demand for more accurate detection of target molecules for such purposes as earlier detection of disease. Non-Patent Literature (NPL) 1 discloses a technique for inducing an enzyme reaction in a large number of micro compartments, and detecting a fluorescence signal, as an example of the technique for accurately detecting a target molecule. This technique is called digital measurement.
In digital measurement, a sample solution is divided into a very large number of microsolutions. Then, a signal from each microsolution is binarized, and the number of target molecules is measured by determining only whether the target molecule is present or not. The digital measurement can significantly improve the detection sensitivity and quantitativeness compared to the conventional real-time PCR technique or the like.
In digital PCR, which is one type of the digital measurement, the mixture of a PCR reaction reagent and a nucleic acid is diluted so that the number of template nucleic acids present in a single microdroplet within a micro compartment is zero to one. In digital PCR, in order to increase the nucleic acid amplification sensitivity and perform nucleic acid amplification simultaneously for a large number of microdroplets, the volume of each microdroplet is preferably small. For example, NPL 1 discloses a method using micrometer-sized droplets formed so that the volume of each well is several nanoliters.
Another example of digital measurement is the digital invasive cleavage assay (ICA). Patent Literature (PTL) 1 discloses a technique for introducing, into a device including micro wells, a sample including DNA, that is, a PCR product obtained by amplifying and denaturing a DNA sample by PCR, and detecting the DNA using an Invader assay without amplifying the DNA at the time of detection. [PTL 2] provides a system, including methods, apparatus and compositions for performing multiplexed assay of Single-Stranded and Double-Stranded Templates.

[Citation List]

[Patent Literature]

   [PTL 1] WO2015/115635 A1
   [PTL 2] US 2014/274786 A1

[Non-Patent Literature]

[NPL 1] Olmedillas-Lopez S., et al., Current and Emerging Applications of Droplet Digital PCR in Oncology. Mol Diagn Ther. 2017 Oct; 21(5): 493-510

[Summary of the Invention]

[Technical Problem]

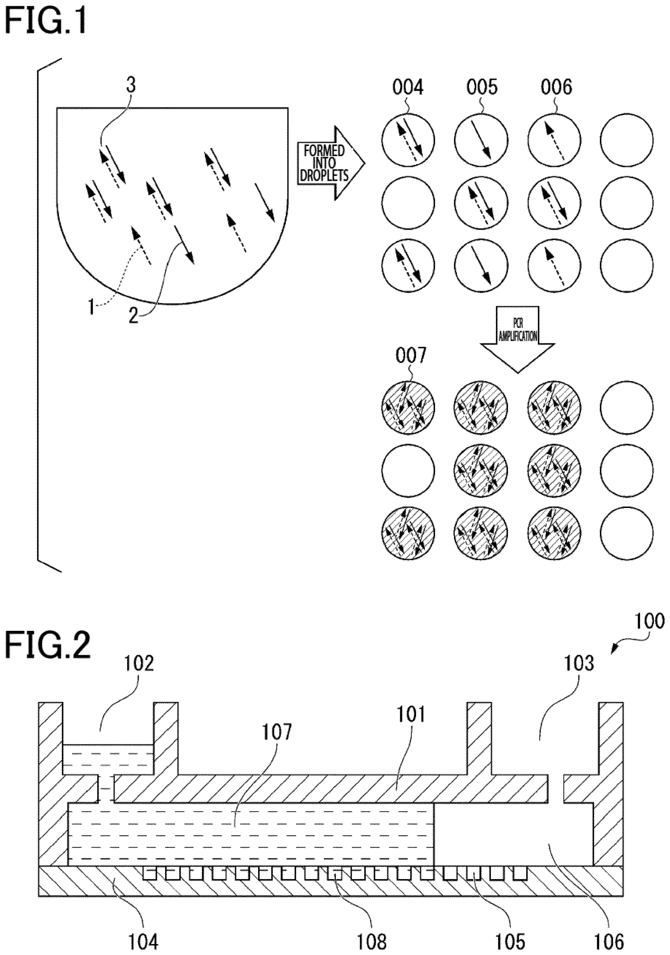

Fig. 1 schematically illustrates a method for detecting a target nucleic acid by digital PCR which is a conventional detection method. When detecting a target nucleic acid by digital PCR, the target nucleic acid is amplified in the step of detecting the target nucleic acid. Therefore, as illustrated in Fig. 1, the target nucleic acid is housed in micro compartments without having been processed through the amplifying step. The target nucleic acid that is DNA, RNA, or the like purified from biological samples such as blood and cells incudes a single-stranded nucleic acid (referred to as a single-stranded nucleic acid 1), a single-stranded nucleic acid that is complementary to the single-stranded nucleic acid 1 (referred to as a single-stranded nucleic acid 2), and a double-stranded nucleic acid resulting from complimentary binding of the single-stranded nucleic acid 1 and the single-stranded nucleic acid 2 (referred to as a double-stranded nucleic acid 3).

For example, as illustrated in Fig. 1, a sample solution that has not yet been formed into droplets includes two molecules of the single-stranded nucleic acid 1, two molecules of the single-stranded nucleic acid 2, and four molecules of the double-stranded nucleic acid 3. For example, when detecting the target nucleic acid using digital PCR which is a conventional detection method, single strand and double-strand are not distinguished from each other while the target nucleic acid is in the form of droplets and are confined as in a droplet 4, a droplet 5, and a droplet 6, for example. PCR products from the droplets 4, 5, 6 of the target nucleic acid are substantially the same. This means that in the digital PCR, eight detection signals are detected from droplets 7, as illustrated in Fig. 1. With this detection result, it is not possible to distinguish whether the sample solution includes eight molecules of the single-stranded nucleic acid 1, eight molecules of the single-stranded nucleic acid 2, or eight molecules of the double-stranded nucleic acid 3. Thus, with the digital PCR method, it is not possible to individually quantify the single-stranded nucleic acid 1, the single-stranded nucleic acid 2, and the double-stranded nucleic acid 3 in the solution.

The present invention has been conceived in view of the above-described circumstances, and has an object to provide a technique that enables more accurate individual quantification of single-stranded nucleic acids and double-stranded nucleic acids.

[Solution to Problem]

The present invention has the following aspects.

[1] A detection method including: bringing liquid including a target nucleic acid into contact with a device including a well array having a plurality of wells, and introducing the target nucleic acid into a well among the plurality of wells in a manner to cause at most one molecule to be included per well; sealing the well to prevent the target nucleic acid from moving between the plurality of wells; amplifying, in the well, a signal derived from the target nucleic acid; and detecting the signal emitted from the well, wherein the target nucleic acid includes a first nucleic acid, a second nucleic acid that is complementary to the first nucleic acid, and a double-stranded nucleic acid resulting from complementary binding of the first nucleic acid and the second nucleic acid, in the sealing, the double-stranded nucleic acid has a double-strand when sealed within the well, in the amplifying of the signal, a first signal is emitted as a result of a first specific binding substance being bound to the first nucleic acid, and a second signal is emitted as a result of a second specific binding substance being bound to the second nucleic acid, and the first signal and the second signal are different.

[2] The detection method described in [1], wherein each of the first signal and the second signal is a luminescence signal, and a wavelength of the first signal and a wavelength of the second signal are different.

[3] The detection method described in [1] or [2], wherein an invasive cleavage assay is used for the amplifying of the signal.

[4] The detection method described in any one of [1] to [3], wherein in the detecting, the well from which only the first signal has been detected is determined as including only the first nucleic acid that is single-stranded, the well from which only the second signal has been detected is determined as including only the second nucleic acid that is single-stranded, and the well from which both the first signal and the second signal have been detected is determined as including the double-stranded nucleic acid.

[5] The detection method described in any one of [1] to [4], wherein the detecting further includes counting, among the plurality of wells, a total number of wells from f which each signal has been detected.

[6] The detection method described in [5], wherein the counting includes counting, among the plurality of wells, a total number of wells from each of which only the first signal has been detected, a total number of wells from each of which only the second signal has been detected, and a total number of wells from each of which both the first signal and the second signal have been detected.

[7] The detection method described in [6], wherein a ratio of a total number of double-stranded nucleic acids each of which is the double-stranded nucleic acid to a sum of a total number of first nucleic acids each of which is the first nucleic acid and is single-stranded and the total number of the double-stranded nucleic acids in the liquid is calculated from the total number of the wells which only the first signal has been detected and the total number of the wells from which both the first signal and the second signal have been detected.

[8] The detection method described in any one of [1] to [7], wherein a difference between a melting temperature Tm of the first specific binding substance and the first nucleic acid and a melting temperature Tm of the second specific binding substance and the second nucleic acid is less than or equal to 10 degrees Celsius.

[9] A detection method including: bringing liquid including a target nucleic acid into contact with a device including a well array having a plurality of wells, and introducing the target nucleic acid into a well among the plurality of wells in a manner to cause at most one molecule to be included per well; sealing the well to prevent the target nucleic acid from moving between the plurality of wells; amplifying, in the well, a signal derived from the target nucleic acid;

and detecting the signal emitted from the well, wherein the target nucleic acid includes a first nucleic acid, a second nucleic acid that is complementary to the first nucleic acid, and a double-stranded nucleic acid resulting from complementary binding of the first nucleic acid and the second nucleic acid, in the sealing, the double-stranded nucleic acid has a double-strand when sealed within the well, and in the amplifying of the signal, a first signal is emitted as a result of a first specific binding substance being bound to the first nucleic acid.

Also disclosed but not part of the invention is a detection method for detecting a target nucleic acid in liquid, the detection method including: bringing the liquid into contact with a device including a well array having a plurality of wells, and introducing the target nucleic acid into a well among the plurality of wells in a manner to cause at most one molecule to be included per well; sealing the well to prevent the target nucleic acid from moving between the plurality of wells; amplifying, in the well, a signal derived from the target nucleic acid; and detecting the signal emitted from the well, wherein the target nucleic acid includes a first nucleic acid and a second nucleic acid that is complementary to the first nucleic acid, in the amplifying of the signal, a first signal is emitted as a result of a first specific binding substance being bound to the first nucleic acid, and a second signal is emitted as a result of a second specific binding substance being bound to the second nucleic acid, and the first signal and the second signal are different.

[11] In one embodiment, each of the first signal and the second signal is a luminescence signal, and a wavelength of the first signal and a wavelength of the second signal are different. In one embodiment, wherein an invasive cleavage assay is used for the amplifying of the signal. In one embodiment, wherein in the detecting, the well from which only the first signal has been detected is determined as including only the first nucleic acid that is single-stranded, the well from which only the second signal has been detected is determined as including only the second nucleic acid that is single-stranded, and the well from which both the first signal and the second signal have been detected is determined as including a double-strand resulting from complementary binding of the first nucleic acid and the second nucleic acid. In one embodiment, the detecting further includes counting, among the plurality of wells, a total number of wells from each of which the signal has been detected.

In one embodiment, the counting includes counting, among the plurality of wells, a total number of wells from each of which only the first signal has been detected, a total number of wells from each of which only the second signal has been detected, and a total number of wells from each of which both the first signal and the second signal have been detected.

In one embodiment, a ratio of the double-strand resulting from the complementary binding of the first nucleic acid and the second nucleic acid to the first nucleic acid that is single-stranded and the double-strand resulting from the complementary binding of the first nucleic acid and the second nucleic acid in the liquid is calculated from the total number of the wells from each of which only the first signal has been detected and the total number of the wells from each of which both the first signal and the second signal have been detected. Also disclosed but not part of the invention is a detection method for detecting a target nucleic acid in liquid, the detection method including: bringing the liquid into contact with a device including a well array having a plurality of wells, and introducing the target nucleic acid into a well among the plurality of wells in a manner to cause at most one molecule to be included per well; sealing the well to prevent the target nucleic acid from moving between the plurality of wells; amplifying, in the well, a signal derived from the target nucleic acid; and detecting the signal emitted from the well, wherein the target nucleic acid includes a first nucleic acid and a second nucleic acid that is complementary to the first nucleic acid, and in the amplifying of the signal, a first signal is emitted as a result of a first specific binding substance being bound to the first nucleic acid.

[Advantageous Effects of the Invention]

With the present invention, it is possible to provide a technique that enables more accurate individual quantification of a single-stranded nucleic acid and a double-stranded nucleic acid.

[Brief Description of the Drawings]

Fig. 1 schematically illustrates a method for detecting a target nucleic acid by digital PCR.
Fig. 2 is a schematic cross-sectional view illustrating a method for supplying a reagent solution to a device.
Fig. 3 is a schematic cross-sectional view illustrating a method for supplying oil to the device.
Fig. 4 is a schematic cross-sectional view illustrating a method for detecting a signal from the device.
Fig. 5 schematically illustrates a detection method according to the present embodiment.

[Description of the Embodiments]

Hereinafter, an embodiment of the present invention will be described in detail with reference to the drawings as appropriate. Note that in the drawings, the same or corresponding parts are denoted by the same or corresponding reference numerals, and redundant description is omitted. Furthermore, the drawings are not necessarily to scale; some of the dimension ratios in the figures are exaggerated for the sake of illustration.

[Detection Method]

In one embodiment, the present invention provides a detection method including: bringing liquid including a target nucleic acid into contact with a device including a well array having a plurality of wells, and introducing the target nucleic acid into a well among the plurality of wells in a manner to cause at most one molecule to be included per well; sealing the well to prevent the target nucleic acid from moving between the plurality of wells; amplifying, in the well, a signal derived from the target nucleic acid; and detecting the signal emitted from the well, wherein the target nucleic acid includes a first nucleic acid, a second nucleic acid that is complementary to the first nucleic acid, and a double-stranded nucleic acid that is a double-strand resulting from complementary binding of the first nucleic acid and the second nucleic acid, in the sealing, the double-stranded nucleic acid has a double-strand when sealed within the well, in the amplifying of the signal, a first signal is emitted as a result of a first specific binding substance being bound to the first nucleic acid, and a second signal is emitted as a result of a second specific binding substance being bound to the second nucleic acid, and the first signal and the second signal are different.

With the detection method according to the present embodiment, by detecting a signal derived from the target nucleic acid, it is possible to distinguish whether each well includes only the first nucleic acid, only the second nucleic acid, or only a double-strand resulting from the complementary binding of the first nucleic acid and the second nucleic acid, as will be described later. As a result, the number of molecules of the first nucleic acid, the second nucleic acid, and the double-stranded nucleic acid resulting from the complementary binding of the first nucleic acid and the second nucleic acid, included in the liquid, can be determined with increased accuracy.

For example, when the target nucleic acid originates from a biological sample, accurately determining the number of molecules of each of the first nucleic acid, the second nucleic acid, and the double-stranded nucleic acid may lead to new findings about the status of cancer, diseases associated with genetic mutations, and the like.

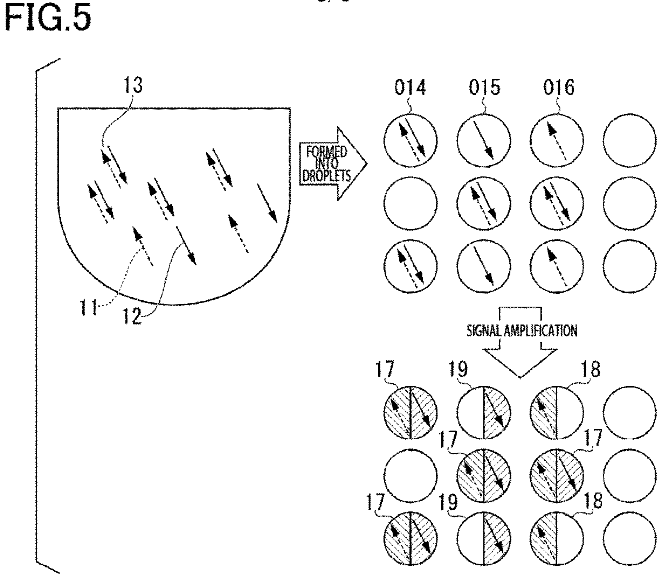

More specifically, as illustrated in Fig. 5, the liquid including the target nucleic acid includes a first nucleic acid 11, a second nucleic acid 12 that is single-stranded and is complementary to the first nucleic acid 11, and a double-stranded nucleic acid 13 that is double-stranded and results from complementary binding of the first nucleic acid 11 and the second nucleic acid 12. The sample solution that has not yet been formed into droplets includes two molecules of the first nucleic acid 11 that is single-stranded, two molecules of the second nucleic acid 12 that is single-stranded, and four molecules of the double-stranded nucleic acid 13. For example, with the detection method according to the present embodiment, the double-stranded nucleic acid 13 is confined in a droplet 14, the second nucleic acid 12 is confined in a droplet 15, the first nucleic acid 11 is confined in a droplet 16, for example, through the introduction step and the sealing step.

Subsequently, in the signal amplifying step, a first signal is emitted from a droplet 18, a second signal is emitted from a droplet 19, and both a first signal and a second signal are emitted from a droplet 17. Since the first signal and the second signal are different, the droplet 17, the droplet 18, and the droplet 19 can be distinguished in the detecting step. This means that with the detection method according to the present embodiment, the first nucleic acid 11, the second nucleic acid 12, and the double-stranded nucleic acid 13 that are included in the droplets can be distinguished. As a result, the liquid including the target nucleic acid can be determined as including two molecules of the first nucleic acid 11, two molecules of the second nucleic acid 12, and four molecules of the double-stranded nucleic acid 13.

(Device)

In the detection method according to the present embodiment, a device including a well array having a plurality of wells is used. Examples of the device include, but are not limited to, the following.

The shape, size, and arrangement of the wells are not especially limited; however, a well array having wells that can accommodate the liquid including the target nucleic acid used in a method according to the present invention and a predetermined amount of the reagent solution or the like used in the detecting step is preferably used. The wells may be used without treatment, or at least one of an extraction reagent, a detection reagent such as an antibody, and a specific binding substance or the like may be immobilized on the inner walls of the wells in advance depending on the purpose. Furthermore, pre-treatment such as covering the opening portions of the wells with a lipid bilayer may be performed.

The device may include a flow path, and liquid including the target nucleic acid dispersed therein may be supplied through the flow path. The shape, structure, capacity, and the like of the flow path are not limited; however, a device including a flow path is preferably used. As a result of use of such a device, when the liquid including the target nucleic acid is supplied, the target nucleic acid is introduced into each well of the well array, and when a sealant is inserted into the flow path, each well is individually sealed, and thus microdroplets can be formed.

(Example of Device)

Fig. 2 is a schematic cross-sectional view of the device according to one aspect of the present invention. As shown in Fig. 2, a device 100 includes a substrate 104 and a cover member 101. The cover member 101 includes a protruding portion. The protruding portion is connected to the substrate 104. The cover member 101 includes a liquid supply port 102 and a liquid discharge port 103 each having a hole penetrating the cover member 101. The substrate 104 has a plurality of wells 105. A flow path 106 is located between the cover member 101 and the plurality of wells 105.

The substrate 104 may be made of a light-transmitting resin. The substrate 104 according to the present embodiment may be substantially transparent.

The wells 105 of the substrate 104 are open to the surface of the substrate 104. The shape, size, and arrangement of the wells 105 are not limited. In the example shown in Fig. 2, in the device 100, a plurality of wells 105 of the same size and shape that accommodate a reagent solution 107 (liquid having the target nucleic acid dispersed therein) are formed in the substrate 104. Furthermore, when particles are used in the detection method according to the present embodiment, the wells 105 that are sized and shaped so as to accommodate one or more particles are of the same size and shape, and can accommodate a predetermined amount of the reagent solution 107 including the particles that may be formed in the substrate 104.

In the device 100, the diameter of the well 105 may be 100 nm to 30 $\mu$m and is preferably in the range of 1 $\mu$m to 15 $\mu$m and more preferably in the range of 3 $\mu$m to 15 $\mu$m. The depth of the well may be 100 nm to 30 $\mu$m and is preferably in the range of 1 $\mu$m to 15 $\mu$m and more preferably in the range of 3 $\mu$m to 15 $\mu$m. As one example, the diameter of the well may be approximately 3 $\mu$m, and the depth of the well 105 may be approximately 4.5 $\mu$m. The wells 105 may be arranged in the form of a triangular lattice or a square lattice in the substrate 104.

The number of wells in the device 100 is preferably in the range of 100,000 to 6,000,000. The total capacity of the wells is preferably in the range of 0.1 $\mu$L to 10 $\mu$L.

A region of the substrate 104 that includes the plurality of wells 105 is a region to be filled with the reagent solution 107, which is a subject of analysis. Inside this region, the flow path 106 is provided between the substrate 104 and the cover member 101.

The cover member 101 may be welded or bonded to the substrate 104. For example, the cover member 101 may be made of a thermoplastic resin such as a cycloolefin polymer or a cycloolefin copolymer.

The substrate 104 is made of, for example, a resin. The type of the resin is not limited, but a resin that is resistant to the reagent and the sealant used in forming droplets is preferably used. Furthermore, for fluorescence observation of a signal, it is preferable to select a resin with low autofluorescence that transmits light of the detection wavelength. The examples of the resin include a cycloolefin polymer, a cycloolefin copolymer, silicone, polypropylene, polycarbonate, polystyrene, polyethylene, polyvinyl acetate, a fluororesin, and an amorphous fluororesin. These example materials of the substrate 104 are merely illustrative, and do not limit the material of the substrate 104.

The plurality of wells 105 may be formed in one surface of the substrate 104 in the thickness direction. The examples of a forming method using the resin include thermal imprinting and optical imprinting as well as injection molding. When a fluororesin is used, for example, a layer of CYTOP (registered trademark) (AGC Inc.) may be formed on the substrate 104, and fine apertures formed in the CYTOP (registered trademark) may serve as the wells 105.

The cover member 101 is formed to have a protruding portion on a surface that faces the substrate 104 when assembled. For example, a fluid of a thermoplastic resin may be molded into a plate shape having a protruding portion by using a mold. In the device 100 shown in Fig. 2, the liquid supply port 102 and the liquid discharge port 103 are formed in the cover member 101, but this is not limiting; at least one of the liquid supply port 102 and the liquid discharge port 103 is not required to be formed.

Once the cover member 101 and the substrate 104 are formed as described above, the cover member 101 and the substrate 104 are stacked with the protruding portion of the cover member 101 in contact with the surface of the substrate 104 to which the wells 105 are open. Furthermore, the cover member 101 and the substrate 104 are welded to each other by laser welding or the like while being stacked as just described.

Hereinafter, each of the steps will be described in detail.

(Introducing Step)

In the present step, the liquid including the target nucleic acid is brought into contact with the device and is introduced into the wells so that at most one molecule of the target nucleic acid is included per well. The wording "introducing" the target nucleic acid into the wells refers to distributing the target nucleic acid into the respective wells of the well array.

More specifically, in the present embodiment, when the device 100 is used, as illustrated in Fig. 2, the reagent solution 107 (in other words, the liquid including the target nucleic acid) diluted so as to allow at most one molecule of the target nucleic acid to be introduced into each of the wells 105 of the device 100 may be supplied from the liquid supply port 102 of the cover member 101 to the flow path 106 located between the cover member 104 and the cover member 101. The reagent solution 107 supplied into the flow path 106 located between the substrate 104 and the cover member 101 is drawn into the plurality of wells 105.

The target nucleic acid may be dispersed in the liquid. Liquid for dispersing the target nucleic acid may be commonly available liquid that is used in biochemical analysis conducted using the above-described device, and is preferably an aqueous solution. The aqueous solution may contain surfactant or the like in order to facilitate sealing of the wells containing the liquid. In addition, a reagent or the like that is necessary, for example, in the step of extracting the target nucleic acid or the step of detecting the target nucleic acid, which will be described later, may also be contained. For example, when an invasive cleavage assay (ICA) reaction is used for detection of the target nucleic acid, ICA reaction reagents such as an allele probe, ICA oligo, flap endonuclease-1 (FEN-1), and a fluorogenic substrate may also be contained in the liquid for dispersing the target nucleic acid.

A means for introducing the target nucleic acid into the wells is not limited, and an appropriate means for the selected target nucleic acid can be used. Alternatively, a substance (capture substance) that captures the target nucleic acid may be used; a target nucleic acid that is unlikely to precipitate by its own weight can be bound to the capture substance when the liquid is supplied, or the capture substance can be immobilized in the wells in advance so as to capture the target nucleic acid in the supplied liquid, allowing for improved introduction efficiency.

In the introducing step, the target nucleic acid is introduced in a manner to cause at most one molecule to be included per well. Specifically, zero or one molecule of the target nucleic acid is introduced into a single well. The phrase "introducing the target nucleic acid in a manner to cause at most one molecule to be included per well" means that every well becomes either a well into which one of one molecule of the first nucleic acid, one molecule of the second nucleic acid, and one molecule of the double-stranded nucleic acid is introduced or a well into which none of the first nucleic acid, the second nucleic acid, and the double-stranded nucleic acid is introduced. Accordingly, the target nucleic acid can be detected on a per piece basis (on a per molecule basis), meaning that digital measurement is possible. Note that the target nucleic acid is not always required to be introduced into every well of the well array.

Specific examples of the target nucleic acid herein include DNA, RAN, miRNA, mRNA and an artificial nucleic acid. The target nucleic acid may be obtained through artificial synthesis or may be obtained by being separated from a biological sample. Examples of the biological sample include human cells, blood, lymph, interstitial fluids, coelomic fluids, digestive fluids, sweat, tears, nasal discharges, urine, semen, vaginal fluids, amniotic fluids, breast milk, and cultured cells.

The length of the target nucleic acid is not limited, but is preferably in the range of 10 bases to 1,000 bases and more preferably in the range of 30 bases to 300 bases. When the target nucleic acid is cfDNA in blood, the length of the target nucleic acid is preferably in the range of 100 bases to 200 bases.

The target nucleic acid may be obtained by fragmenting a nucleic acid chain separated from a biological sample. The nucleic acid chain can be fragmented using, for example, a DNA fragmentation device (for example, Covaris manufactured by M&S Instruments Inc.).

The target nucleic acid may be a nucleic acid sequence known to relate to disease. For example, the target nucleic acid may be a nucleic acid sequence including a region known to have a mutation in a cancer patient. Specific examples of the target nucleic acid include a portion of the base sequence at the human epidermal growth factor receptor (EGFR) locus and a portion of the base sequence at the human vascular endothelial growth factor (VEGF) locus. One example of a mutation that occurs in a portion of the base sequence at the human epidermal growth factor receptor (EGFR) locus is T790M.

In the present embodiment, the target nucleic acid includes the first nucleic acid and the second nucleic acid that is complementary to the first nucleic acid. The sequence of the first nucleic acid and the sequence of the second nucleic acid may or may not be entirely complementary to each other. For example, there may be a mismatch in 1% to 20% of the bases in the entire base sequence of the longer strand of the sequence of the first nucleic acid and the sequence of the second nucleic acid.

The liquid including the target nucleic acid may include the first nucleic acid that is single-stranded, the second nucleic acid that is single-stranded, and the double-stranded nucleic acid resulting from the complementary binding of the first nucleic acid and the second nucleic acid.

The double-stranded nucleic acid may have a double-strand that are completely complementary or may include sequences that are not complementary. For example, there may be a mismatch in 1% to 20% of the bases in the entire base sequence of the longer strand of the double-stranded nucleic acid. The double-stranded nucleic acid may have double-strand of the same or different types among DNA, RNA, miRNA, mRNA, and an artificial nucleic acid. Examples of the double-strand of different types include double-strand composed of a DNA strand and an RNA strand and double-strand composed of a DNA strand and an artificial nucleic acid strand.

In the present embodiment, the wording "one molecule of the first nucleic acid" refers to one first nucleic acid that is single-stranded, and the wording "one molecule of the second nucleic acid" refers to one second nucleic acid that is single-stranded. The wording "the double-stranded nucleic acid resulting from complementary binding of one molecule of the first nucleic acid and the second nucleic acid" refers to one double-stranded nucleic acid resulting from complementary binding of the first nucleic acid and the second nucleic acid.

(Sealing Step)

In the present step, the plurality of wells are sealed so that the target nucleic acid does not move between the wells. The means for sealing is not limited; for example, a layer of the sealant may be formed on the top layer of the liquid introduced into the wells, the liquid may be encapsulated in the wells, and thus microdroplets may be formed in the wells.

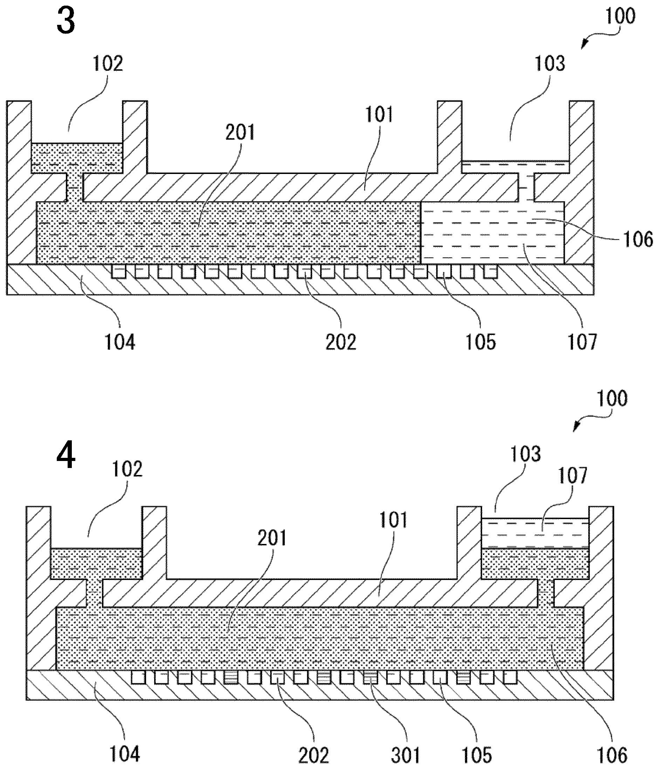

For example, more specifically, in the present embodiment, when the device 100 is used, a sealant 201 such as oil is supplied from the liquid supply port 102 of the cover member 101 into the flow path 106 located between the substrate 104 and the cover member 101 to individually seal the plurality of wells 105, as shown in Fig. 3. In the sealing step, the sealant 201 replaces the reagent solution 107 that has been supplied into the flow path 106 located between the substrate 104 and the cover member 101 in the above-described liquid supplying step, but has not been drawn into the wells 105. Thus, the sealant 201 individually seals the plurality of wells 105, and the wells 105 become independent reaction spaces (micro compartments 202).

At most one molecule of the target nucleic acid is present in each of the wells 105 after the sealing step. In the well 105 in which the double-stranded nucleic acid is present as the target nucleic acid, the double-stranded nucleic acid has double-strand without dissociation into single strand by denaturation when the well is sealed.

The aforementioned sealant 201 is fluid that can individually seal the plurality of wells to prevent the liquids (reagent solutions 107) introduced thereto from being mixed with each other and form liquid droplets (microdroplets); the sealant is preferably an oil-based solution and is more preferably an oil. Examples of the oil include fluorine-based oil, silicone-based oil, hydrocarbon-based oil, and a mixture thereof; for example, the oil manufactured by SIGMA CORPORATION under the product name "FC-40" can be used.

The device used in the present embodiment is not required to include the flow path as long as the plurality of wells can be sealed so that the target nucleic acid does not move between the wells. In the case where this device is used, for example, each well filled with the liquid including the target nucleic acid may be sealed with the liquid by oil dripped from above.

The device used in the present embodiment may be a micro well plate including neither the cover member nor the flow path as long as the target nucleic acid can be prevented from moving between the plurality of wells. In the case where this device is used, for example, a plate member may be placed in close contact with the wells from above so that the opening portions of the wells are sealed after the liquid including the target nucleic acid is introduced into the wells.

In the case where the micro well plate including neither the cover member nor the flow path is used, in order to prevent the target nucleic acid from moving between the plurality of wells, excess liquid on the well plate may be removed after the liquid including the target nucleic acid is introduced into the wells. For example, the excess liquid on the well plate may be removed using a squeegee or may be removed by suction using a suction device.

The double-stranded nucleic acid present in the individually sealed well may be denatured after the sealing step and before the signal amplifying step. The conditions of denaturation may be set, as appropriate, according to the heat resistance of enzymes used in the signal amplifying step. The denaturation temperature may be in the range of 55°C to 99°C and preferably in the range of 70°C to 99°C. The temperature rise time to the denaturation temperature may be in the range of 25 seconds to 90 seconds and preferably in the range of 30 seconds to 60 seconds. The time of retention at the denaturation temperature may be in the range of 10 seconds to 40 seconds and preferably in the range of 30 seconds to 40 seconds.

The denaturation temperature, the temperature rise time to the denaturation temperature, and the time of retention that are included in the conditions of denaturation can be arbitrarily combined within the aforementioned ranges. For example, the temperature may be increased for 25 seconds to 90 seconds from a room temperature (for example, 25°C) to a denaturation temperature in the range of 55°C to 99°C, and maintained at the denatured temperature for 10 seconds to 40 seconds, to denature the double-stranded nucleic acid.

In the case where the target nucleic acid can be heated to denature the double-stranded nucleic acid in the signal amplifying step, which will be described later, the double-stranded nucleic acid does not need to be denatured before the signal amplifying step.

(Signal Amplifying Step)

In the present step, the signal derived from the target nucleic acid is amplified in the well up to a level at which the signal derived from the target nucleic acid can be detected.

For example, in the present embodiment, when the device 100 is used, a reaction that amplifies the signal in the well 105 proceeds, and the well 105 including the target nucleic acid is sealed to contain a signal-emitting microsolution 301, as illustrated in Fig. 4.

The signal that is amplified in the present step is not especially limited; examples of the signal include fluorescence, chemiluminescence, color development, change in electric potential, and change in pH.

The signal amplification reaction may be, for example, a biochemical reaction, and more specifically, an enzymatic reaction. As an example, the signal amplification reaction may be an isothermal reaction in which a device accommodating, in wells, a reagent solution including an enzyme for signal amplification is maintained under a constant temperature condition in which a desired enzyme activity can be obtained. This constant temperature condition is that the device is to be maintained in the range of 60°C to 99°C, preferably at about 66°C, for example, for at least 10 minutes, preferably about 15 minutes, for example.

Specific examples of the signal amplification reaction include ICA reactions such as an Invader (registered trademark) assay.

Use of the ICA reaction as the signal amplification reaction is especially preferable (for example, refer to WO2009/054474 A1). This is related to the principle of the ICA reaction that the signal amplification proceeds by the cycle of the following two reactions: (1) complementary binding of nucleic acids; and (2) recognition and cleavage of a triple-stranded structure by an enzyme. In such a signal amplification reaction, inhibitory influence that contaminants other than the target nucleic acid have on the reaction cycle is small. Therefore, even if various components other than the target nucleic acid are present in the micro compartments, the target nucleic acid can be accurately detected by using the ICA reaction. For example, when the ICA reaction is used for the signal amplification reaction, liquid for introducing the target nucleic acid into the wells (liquid for dispersing the target nucleic acid) includes the target nucleic acid and a reaction reagent required for the ICA reaction. When the biochemical reaction in the signal amplifying step is the ICA reaction, and the target nucleic acid is present in the well as a result of an enzymatic reaction due to the isothermal reaction, a fluorescent substance is released from a quenching substance, causing emission of a predetermined fluorescence signal corresponding to excitation light.

Alternatively, the target nucleic acid can also be detected by way of binding, to the target nucleic acid, a substance that is sequence-specifically bound to the target nucleic acid (specific binding substance), and detecting the specific binding substance that has been bound.

Examples of the specific binding substance include those similar to the specific binding substance for the target nucleic acid, which will be described later, such as antibodies, antibody fragments, polypeptides, and aptamers. When the signal amplifying step is the ICA reaction, a FLAP probe, invader probe, and the like can be used as the specific binding substance. As the FLAP probe and invader probe, those prepared by known methods can be used so that the first nucleic acid and the second nucleic acid can be identified.

The difference between Tm (also referred to as the melting temperature) of the first specific binding substance and the first nucleic acid and Tm of the second specific binding substance and the second nucleic acid is preferably less than or equal to 10°C. When the difference between Tm of the first specific binding substance and the first nucleic acid and Tm of the second specific binding substance and the second nucleic acid is less than or equal to 10°C, both the first nucleic acid and the second nucleic acid can be detected in an isothermal reaction such as the ICA reaction.

In the present step, the first signal derived from the first nucleic acid and the second signal derived from the second nucleic acid are amplified. The first signal and the second signal are different. More specifically, when the biochemical reaction in the signal amplifying step is the ICA reaction, the FLAP probe and the invader probe for the first nucleic acid are bound to the first nucleic acid that is single-stranded or the first nucleic acid resulting from dissociation of the double-stranded nucleic acid. The FLAP probe and the invader probe for the second nucleic acid are bound to the second nucleic acid that is single-stranded or the second nucleic acid resulting from dissociation of the double-stranded nucleic acid.

The first signal and the second signal may be luminescence signals. When the first signal and the second signal are luminescence signals, the wavelength of the first signal and the wavelength of the second signal are different. More specifically, when the signal amplifying step is the ICA reaction, the wavelength of the first signal and the wavelength of the second signal can be made different by using two different fluorescent substances that produce fluorescence emissions. Specifically, by using the first fluorescent substance for detecting the first nucleic acid and the second fluorescent substance for detecting the second nucleic acid that produces fluorescence of a wavelength different from the wavelength of the first fluorescent substance, the first signal and the second signal can be amplified respectively.

(Detecting Step)

In the present step, the signal amplified in the signal amplifying step is detected. A signal detection method may be selected from known appropriate methods according to the type of the signal to be detected. For example, in the case where the detection is performed in a bright field, white light is emitted perpendicularly to the substrate provided with the well array, for example. In the case of detecting a fluorescence signal, for example, excitation light corresponding to the fluorescent substance is emitted into the well via the bottom of the well, and fluorescence emitted by the fluorescent substance is detected. In the present step, for example, an image of a portion or the entirety of the well array may be captured and stored, and the image may be processed using a computer system.

In the present embodiment, as described above regarding the introducing step, the target nucleic acid included in each well is only one molecule of the first nucleic acid, only one molecule of the second nucleic acid, or only one molecule of the double-stranded nucleic acid resulting from complementary binding of the first nucleic acid and the second nucleic acid. Furthermore, there may be a well that does not include the target nucleic acid.

Therefore, the result of detection from each well is the first signal only, the second signal only, both the first signal and the second signal, or no signal at all.

In other words, the well from which only the first signal has been detected can be determined as including only the first nucleic acid that is single-stranded, the well from which only the second signal has been detected can be determined as including only the second nucleic acid that is single-stranded, and the well from which both the first signal and the second signal have been detected can be determined as including the double-stranded nucleic acid resulting from complementary binding of the first nucleic acid and the second nucleic acid.

(Counting Step)

The detection method according to the present embodiment may further include a counting step in which the number of wells from each of which the signal has been detected is counted.

More specifically, in the counting step, the number of wells from each of which only the first signal has been detected, the number of wells from each of which only the second signal has been detected, and the number of wells from each of which both the first signal and the second signal have been detected may be counted.

Thus, it is possible to calculate the number of wells in each of which only the first nucleic acid that is single-stranded is present, the number of wells in each of which only the second nucleic acid that is single-stranded is present, and the number of wells in each of which the double-stranded nucleic acid resulting from complementary binding of the first nucleic acid and the second nucleic acid is present.

Accordingly, it is possible to calculate the ratio of the double-strand resulting from the complementary binding of the first nucleic acid and the second nucleic acid to the first nucleic acid that is single-stranded and the double-stranded nucleic acid resulting from complementary binding of the first nucleic acid and the second nucleic acid in the liquid including the target nucleic acid distributed to the wells.

In the method for detecting the target nucleic acid in the liquid according to the present embodiment, an integrated apparatus may be used that includes: a device into which the target nucleic acid is introduced; a light source that is used in the detecting step; and a detector that detects the signal. This device may further include a processor that processes an image, etc., of the detected signal and calculates the aforementioned ratio of the target nucleic acid.

In the method for detecting the target nucleic acid in the liquid according to the present embodiment, a system may be used that includes: a housing apparatus that supports a device into which the target nucleic acid is introduced; a light source apparatus that is used in the detecting step; and a detection apparatus that detects the signal. This system may further include a processor that processes an image, etc., of the detected signal and calculates the aforementioned ratio of the target nucleic acid.

In one embodiment, the present invention provides a detection method including: bringing liquid including a target nucleic acid into contact with a device including a well array having a plurality of wells, and introducing the target nucleic acid into a well among the plurality of wells in a manner to cause at most one molecule to be included per well; sealing the well to prevent the target nucleic acid from moving between the plurality of wells; amplifying, in the well, a signal derived from the target nucleic acid; and detecting the signal emitted from the well, wherein the target nucleic acid includes a first nucleic acid, a second nucleic acid that is complementary to the first nucleic acid, and a double-stranded nucleic acid resulting from complementary binding of the first nucleic acid and the second nucleic acid, in the sealing, the double-stranded nucleic acid has double-strand when sealed within the well, and in the amplifying of the signal, a first signal is emitted as a result of a first specific binding substance being bound to the first nucleic acid.

With the detection method according to the present embodiment, by detecting a signal derived from the first nucleic acid, it is possible to distinguish whether or not each well includes the first nucleic acid, as will be described later in an example. The well including the first nucleic acid includes either the first nucleic acid that is single-stranded or the double-strand resulting from the complementary binding of the first nucleic acid and the second nucleic acid. As a result, it is possible to accurately determine the total number of molecules of the first nucleic acid that is single-stranded and the total number of molecules of the double-stranded nucleic acid resulting from complementary binding of the first nucleic acid and the second nucleic acid in the liquid, as will be described later in the example.

Also disclosed but not part of the invention:

[18] A detection method comprising: bringing liquid including a first nucleic acid, a second nucleic acid that is complementary to the first nucleic acid, and a double-stranded nucleic acid resulting from complementary binding of the first nucleic acid and the second nucleic acid as a target nucleic acid into contact with a device including a well array having a plurality of wells, and introducing the target nucleic acid into a well among the plurality of wells in a manner to cause at most one molecule to be included per well; sealing the well to prevent the target nucleic acid from moving between the plurality of wells; denaturing the double-stranded nucleic acid after the sealing; amplifying, in the well, a signal derived from the target nucleic acid; and detecting the signal emitted from the well, wherein in the sealing, the double-stranded nucleic acid has a double-strand when sealed within the well, in the amplifying of the signal, a first signal is emitted as a result of a first specific binding substance being bound to the first nucleic acid, and a second signal is emitted as a result of a second specific binding substance being bound to the second nucleic acid, and the first signal and the second signal are different.

[19] In one embodiment, each of the first signal and the second signal is a luminescence signal, and a wavelength of the first signal and a wavelength of the second signal are different.

[20] In one embodiment, an invasive cleavage assay is used for the amplifying the signal.

[21] In one embodiment, in the detecting, the well from which only the first signal has been detected is determined as including only the first nucleic acid that is single-stranded, the well from which only the second signal has been detected is determined as including only the second nucleic acid that is single-stranded, and the well from which both the first signal and the second signal have been detected is determined as including the double-stranded nucleic acid.

[22] In one embodiment, the detecting further includes counting, among the plurality of wells, a total number of wells from each of which the signal has been detected.

[23] In one embodiment, the counting includes counting, among the plurality of wells, a total number of wells from each of which only the first signal has been detected, a total number of wells from each of which only the second signal has been detected, and a total number of wells from each of which both the first signal and the second signal have been detected.

[24] In one embodiment, a ratio of a total number of double-stranded nucleic acids each of which is the double-stranded nucleic acid to a sum of a total number of first nucleic acids each of which is the first nucleic acid and is single-stranded and the total number of the double-stranded nucleic acids in the liquid is calculated from the total number of the wells from each of which only the first signal has been detected and the total number of the wells from each of which both the first signal and the second signal have been detected.

[25] In one embodiment, a difference between a melting temperature Tm of the first specific binding substance and the first nucleic acid and a melting temperature Tm of the second specific binding substance and the second nucleic acid is less than or equal to 10 degrees Celsius.

[26] In one embodiment, the amplification of the signal is an isothermal reaction.

[27] In one embodiment, the denaturing and the amplification of the signal are performed simultaneously.

[28] In one embodiment, the denaturing is performed at a temperature greater than a temperature at which the amplification of the signal is performed.

[29] In one embodiment, the denaturing is performed at a temperature greater than a temperature at which the amplification of the signal is performed.

[30] In one embodiment, the denaturing includes retention at a temperature of 55 degrees Celsius to 99 degrees Celsius for 10 seconds to 40 seconds.

[Examples]

The present invention will be described below by way of examples, but the present invention should not be limited to these examples.

[Experimental Example 1]

(Detection of Nucleic Acid in Cell by ICA Method)

A region including a part of the base sequence at the human epidermal growth factor receptor (EGFR) locus was set as the target nucleic acid, and the target nucleic acid was quantitatively detected. A part of the region of the human EGFR is known to have a mutation in some cancer patients. In the present experimental example, the sense strand (SEQ ID NO: 7) at the EGFR locus was selected as the target nucleic acid.

<Preparation of DNA Sample>

A genome DNA was separated from a cultured human cell (HT29) using a DNA extraction kit (AllPrep manufactured by QIAGEN), and the separated DNA was fragmented using a DNA fragmentation device (Covaris manufactured by M&S Instruments Inc.) so as to provide simulated circulating DNA specimens in the blood; thus, a DNA sample in Preparation Example 1 was made.

In order to measure the concentration of the fragmented DNA sample, the DNA sample was rapidly cooled after thermal denaturation at 95°C for 10 minutes, and thus a single-stranded DNA sample was made. The DNA sample immediately after the rapid cooling is predominantly single-stranded. Subsequently, using a microvolume spectrometer (NanoDrop manufactured by Thermo Fisher Scientific Inc.), the absorbance of the DNA sample after the rapid cooling was measured, and it was confirmed that the concentration of the single-stranded DNA sample was 1.1 fM. This means that if the DNA sample is entirely double-stranded, the concentration thereof would be 0.55 fM.

The concentration of the target nucleic acid that is single-stranded means the concentration based on the sum of the number of sense strands at the EGFR locus and the number of antisense strands at the EGFR locus. The concentration of the target nucleic acid that is double-stranded means the concentration based on the number of combinations of sense and antisense strands at the EGFR locus. For example, one diploid cell includes four target nucleic acids when every sample is single-stranded, and two target nucleic acids when every sample is double-stranded.

In calculating the aforementioned concentration, first, the mass/volume concentration of the DNA was calculated from the value of the absorbance measured by the NanoDrop. Subsequently, assuming that 3 pg of the DNA sample includes one sense strand at the EGFR locus and one antisense strand at the EGFR locus, the concentration of the single-stranded target nucleic acid included in the DNA sample after the rapid cooling was calculated.

<Preparation of Nucleic Acid Detection Reagent>

In order to detect the nucleic acid by the ICA reaction, an ICA reaction reagent was prepared as a nucleic acid detection reagent. The ICA reaction reagent in the present example includes 0.5 $\mu$M of allele probe 1 (SEQ ID NO: 1) (FLAP probe), 0.1 $\mu$M of invader oligo 1 (SEQ ID NO: 2) (invader probe, also referred to as ICA oligo) (these are manufactured by Fasmac Co., Ltd.), 4 $\mu$M of FRET Cassette (Alexa488-BHQ) (SEQ ID NO: 3) (manufactured by Japan Bio Services Co., Ltd.) (fluorescent substrate), 50 mM of Tris-HCl (pH 7.9), 20 mM of $MgCl_2$, and 0.05 mg/mL of FEN-1. Note that the concentration of each of these components in the ICA reaction reagent is the final concentration in the mixture of the DNA sample and the ICA reaction reagent according to Experimental Example 1. The allele probe 1 and the invader oligo 1, which are used for the ICA reaction, both specifically recognize the nucleotide strand of one of the sense and antisense strands at the EGFR locus.

<Preparation of Device>

A substrate provided with a large number of microwells was produced from a cycloolefin polymer (COP), and a cover member made of COP was attached thereto; thus, the device was prepared. The total volume of the well per square centimeter is 0.93 $\mu$L. The total number of wells used for the measurement was 1,000,000.

<Supply of Reaction Mixed Solution>

The device was supplied with 8 $\mu$l of a solution obtained by mixing the DNA sample in Preparation Example 1 and the aforementioned ICA reaction reagent, and the solution was introduced into the wells. Subsequently, 200 $\mu$l of FC-40 (manufactured by SIGMA CORPORATION) was supplied as a sealant to seal the wells.

Thus, each well was sealed to contain at most one molecule of the DNA. Specifically, each well includes, as the target nucleic acid, only one kind selected from the single-stranded first nucleic acid to which the allele probe 1 is complementarily bound, the single-stranded second nucleic acid that is a complementary strand for the first nucleic acid, and double-strand resulting from complementary binding of the first nucleic acid and the second nucleic acid, or no target nucleic acid.

<Nucleic Acid Detection Reaction>

The aforementioned device that has been supplied with the reaction mixed solution was set on a hot plate, and allowed to react at 66°C for 25 minutes. As a result, recognition of an EGFR gene region by the allele probe and the invader oligo, cleavage of the allele probe by FEN-1, binding of the released allele probe fragment to the FRET Cassette, and cleavage of FRET Cassette by FEN-1 proceeded, and a fluorescence signal was emitted from Alexa488.

<Fluorescence Observation in Well>

After heating at 66°C for 25 minutes, a fluorescence image of the fluorescence signal obtained by a nucleic acid detection reaction in the wells of the device was captured with a fluorescence microscope (BZ-700 manufactured by KEYENCE Corporation) using a 4x objective lens and a GFP fluorescence filter. The exposure time was set to 3,000 msec. Thus, the fluorescence signal derived from the target nucleic acid can be observed in the wells in which the target nucleic acid having the EGFR gene region containing a single nucleotide polymorphism is present. As a result of the observation through the fluorescence microscope, it was confirmed that 13.7 wells per square centimeter produced fluorescence emissions.

<Calculation of Concentration>

With this result, the concentration of the DNA that is complementary to the allele probe and contains the single nucleotide polymorphism can be calculated as follows. Note that the liquid introduced into the wells of the device is a solution obtained by 20 times dilution of a sample measured using the NanoDrop.

$$\text{Concentration} = (13.7 \text{ wells/cm}^2) \div (6.02 \times 10^{23}) \div 0.93 \ \mu\text{L/cm}^2 \times 10^6 \times 20$$

$$= 49 \times 10^{-17} \ (\text{M})$$

$$= 0.49 \ (\text{fM})$$

Specifically, in the DNA sample in Preparation Example 1, the sum of the concentration of the single-stranded first nucleic acid to which the allele probe 1 is complementarily bound and the concentration of the double-strand resulting from the complementary binding of the first nucleic acid and the second nucleic acid was calculated to be 0.49 fM. This is substantially the same as the concentration of the target nucleic acid included in the DNA sample in Preparation Example 1 (0.55 fM) based on the result of the measurement by the NanoDrop.

[Comparative Example 1]

<Detection of Nucleic Acid by Digital PCR Method>

Using ddPCR QX100 and PrimePCR for ddPCR EGFR T790M (manufactured by Bio-Rad Laboratories, Inc), the concentration of the DNA sample was measured by following the procedure set forth in the manual. The DNA sample used is the same as the DNA sample in Preparation Example 1 that was used in Experimental Example 1.

Each well was sealed to contain at most one molecule of the DNA, as in Experimental Example 1 described above. Specifically, each well included, as the target nucleic acid, only one kind selected from the first nucleic acid, the single-stranded second nucleic acid that is a complementary strand for the first nucleic acid, and double-strand resulting from complementary binding of the first nucleic acid and the second nucleic acid, or no target nucleic acid.

In digital PCR, signals are detected from all of the wells including only the single-stranded first nucleic acid, the wells including only the single-stranded second nucleic acid that is a complementary strand for the first nucleic acid, and the wells including only the double-strand resulting from the complementary binding of the first nucleic acid and the second nucleic acid.

As a result of the measurement by the digital PCR, in the DNA sample in Preparation Example 1, the sum of the concentration of the single-stranded first nucleic acid, the concentration of the single-stranded second nucleic acid, and the concentration of the double-strand resulting from the complementary binding of the single-stranded first and second nucleic acids was calculated to be 1 fM.

From the result using the NanoDrop, it was confirmed that the total concentration of the predominantly single-stranded target nucleic acid included in the DNA sample in Preparation Example 1 was 1.1 fM. Furthermore, from the result of Comparative Example 1, the sum of the concentration of the single-stranded first nucleic acid, the concentration of the single-stranded second nucleic acid, and the concentration of the double-strand resulting from the complementary binding of the single-stranded first and second nucleic acids was 1 fM. This result showed that the target nucleic acid included in the DNA sample in Preparation Example 1 was mostly single-stranded.

Meanwhile, the result of Experimental Example 1 showed that the concentration of the single-stranded first nucleic acid to which the allele probe 1 is complementarily bound was 0.49 fM. Thus, it was clear that in the DNA sample in Preparation Example 1, the concentration of the first nucleic acid was about half of the total concentration of the target nucleic acid that is mostly single-stranded.

[Experimental Example 2]

The sense strand at the EGFR locus and one antisense strand at the EGFR locus described in Experimental Example 1 were selected as the target nucleic acid, and the target nucleic acid was quantitatively detected.

<Preparation of DNA Sample>

A genome DNA was extracted from a cultured human cell (HT29) using a DN extraction kit (AllPrep manufactured by QIAGEN). Using a consignment service (DNA shearing service) provided by M&S Instruments Inc., the extracted genome DNA was sheared to 150 bp. A centrifugal dryer (DNA SpeedVac manufactured by Thermo Fisher Scientific Inc.) was used to evaporate liquid from 1.3 ml of a solution of the sheared genome DNA, and then 100 $\mu$l of distilled water was added thereto; thus, a DNA sample in Preparation Example 2 was made.

The absorbance of the DNA sample was measured, and it was confirmed that the concentration of the single-stranded DNA sample was 1.0 fM. This means that if the DNA sample is entirely double-stranded, the concentration thereof would be 0.5 fM.

<Preparation of Nucleic Acid Detection Reagent>

In order to detect the nucleic acid by the ICA reaction, an ICA reaction reagent was prepared as a nucleic acid detection reagent. The ICA reaction reagent in the present example includes 0.5 $\mu$M of allele probe 1 (SEQ ID NO: 1) (FLAP probe), 0.1 $\mu$M of invader oligo 1 (SEQ ID NO: 2) (invader probe, also referred to as ICA oligo), 0.2 $\mu$M of allele probe 2 (SEQ ID NO: 4) (FLAP probe), 5 nM of invader oligo 2 (SEQ ID NO: 5) (invader probe) (these are manufactured by Fasmac Co., Ltd.), 4 $\mu$M of FRET Cassette (Alexa488-BHQ) (SEQ ID NO: 3) (manufactured by Japan Bio Services Co., Ltd.) (fluorescent substrate), 2 $\mu$M of FRET Cassette (Redmond Red-Epoch Eclipse Quencher) (SEQ ID NO: 6) (manufactured by TSUKUBA OLIGO SERVICE CO., LTD) (fluorescent substrate), 50 mM of Tris-HCl (pH 7.9), 20 mM of $MgCl_2$, and 0.05 mg/mL of FEN-1. Note that the concentration of each of these components in the ICA reaction reagent is the final concentration in the mixture of the DNA sample and the ICA reaction reagent according to Experimental Example 2. The allele probe 1, the invader oligo 1, the allele probe 2, and the invader oligo 2 are used for the ICA reaction. The allele probe 1 and the invader oligo 1 specifically recognize the nucleotide strand of one of the sense and antisense strands at the EGFR locus, and the allele probe 2 and the invader oligo 2 specifically recognize the nucleotide strand of the other of the sense and antisense strands at the EGFR locus.

<Preparation of Device and Reaction Mixed Solution>

Using the device described in Experimental Example 1, the reaction mixed solution was supplied in accordance with the same procedure as in Experimental Example 1.

<Nucleic Acid Detection Reaction>

The aforementioned device that has been supplied with the reaction mixed solution was set on a hot plate, and allowed to react at 66°C for 25 minutes. As a result, recognition of an EGFR gene region by the allele probe and the invader oligo, cleavage of the allele probe by FEN-1, binding of the released allele probe fragment to the FRET Cassette, and cleavage of FRET Cassette by FEN-1 proceeded, and a fluorescence signal was emitted from the Alexa488 and Redmond Red.

<Fluorescence Observation in Well>

As a result of capturing a fluorescence image of the fluorescence signal obtained by a nucleic acid detection reaction in the wells of the device under the same condition as in Experimental Example 1, the number of wells from each of which only the fluorescence signal from the Alexa488 was detected was 508, the number of wells from each of which only the fluorescence signal from the Redmond Red was detected was 429, and the number of wells from each of which the fluorescence signals from both the Alexa488 and the Redmond Red were detected was 3.

The well from which only the fluorescence signal from the Alexa488 has been detected represents a well in which only one of the sense and antisense strands at the EGFR locus is present. The well from which only the fluorescence signal from the Redmond Red has been detected represents a well in which only the other of the sense and antisense strands at the EGFR locus is present. The well from which the fluorescence signals from both the Alexa488 and the Redmond Red have been detected represents a well in which a double-stranded nucleic acid having the sense and antisense strands at the EGFR locus is present at the time of sealing the well.

Note that the well from which the fluorescence signals from both the Alexa488 and the Redmond Red have been detected may be one well accidentally sealed with both the sense and antisense strands at the EGFR locus; however, considering that the total number of wells of the device is 924,890 and among those, the number of wells from which the fluorescence signals have been detected is the aforementioned number, such accidental sealing would be very unlikely. In other words, the well from which the fluorescence signals from both the Alexa488 and the Redmond Red have been detected is not one well accidentally sealed with both the sense and antisense strands at the EGFR locus, but is a well in which a double-stranded nucleic acid having the sense and antisense strands of the EGFR locus is present at the time of sealing the well.

The result of Experimental Example 2 shows that when not only the probe for the first nucleic acid, but also the probe for the second nucleic acid are designed and used as appropriate, it is possible to individually detect the wells including the first nucleic acid that is single-stranded, the second nucleic acid that is single-stranded, and the double-strand resulting from the complementary binding of the first nucleic acid and the second nucleic acid. With this result, it is clear that each of the concentration and the number of molecules can be determined in the calculation method described in Experimental Example 1.

[Industrial Applicability]

With the present invention, it is possible to provide a technique that enables more accurate individual quantification of a single-stranded nucleic acid and a double-stranded nucleic acid.

[Reference Signs List]

| | |
|---|---|
| 1 | Single-stranded nucleic acid |
| 2 | Single-stranded nucleic acid |
| 3 | Double-stranded nucleic acid |
| 4, 5, 6, 7 | Droplet |
| 11 | First nucleic acid |
| 12 | Second nucleic acid |
| 13 | Double-stranded nucleic acid |
| 14, 15, 16, 17, 18, 19 | Droplet |
| 100 | Device |
| 101 | Cover member |
| 102 | Liquid supply port |
| 103 | Liquid discharge port |
| 104 | Substrate |
| 105 | Well |
| 106 | Flow path |
| 107 | Reagent solution |
| 108 | Reagent solution filling wells |
| 201 | Sealant |
| 202 | Micro compartment |
| 301 | Signal-emitting micro solution |

**Claims**

1. A detection method comprising:

   bringing liquid including a target nucleic acid into contact with a device including a well array having a plurality of wells, and
   introducing the target nucleic acid into a well among the plurality of wells in a manner to cause at most one molecule to be included per well;
   sealing the well to prevent the target nucleic acid from moving between the plurality of wells;
   amplifying, in the well, a signal derived from the target nucleic acid; and
   detecting the signal emitted from the well,
   wherein
   the target nucleic acid includes a first nucleic acid, a second nucleic acid that is complementary to the first nucleic acid, and a double-stranded nucleic acid resulting from complementary binding of the first nucleic acid and the second nucleic acid,
   in the sealing, the double-stranded nucleic acid has a double-strand when sealed within the well,
   in the amplifying of the signal, a first signal is emitted as a result of a first specific binding substance being bound to the first nucleic acid, and a second signal is emitted as a result of a second specific binding substance being bound to the second nucleic acid, and
   the first signal and the second signal are different.

2. The detection method according to claim 1, wherein

   each of the first signal and the second signal is a luminescence signal, and
   a wavelength of the first signal and a wavelength of the second signal are different.

3. The detection method according to claim 1 or 2, wherein an invasive cleavage assay is used for the amplifying of the signal.

4. The detection method according to any one of claims 1 to 3, wherein

   in the detecting, the well from which only the first signal has been detected is determined as including only the first nucleic acid that is single-stranded,
   the well from which only the second signal has been detected is determined as including only the second nucleic acid that is single-stranded, and
   the well from which both the first signal and the second signal have been detected is determined as including the double-stranded nucleic acid.

5. The detection method according to any one of claims 1 to 4, wherein the detecting further includes counting, among the plurality of wells, a total number of wells from each of which the signal has been detected.

6. The detection method according to claim 5, wherein the counting includes counting, among the plurality of wells, a total number of wells from each of which only the first signal has been detected, a total number of wells from each of which only the second signal has been detected, and a total number of wells from each of which both the first signal and the second signal have been detected.

7. The detection method according to claim 6, wherein a ratio of a total number of double-stranded nucleic acids each of which is the double-stranded nucleic acid to a sum of a total number of first nucleic acids each of which is the first nucleic acid and is single-stranded and the total number of the double-stranded nucleic acids in the liquid is calculated from the total number of the wells from each of which only the first signal has been detected and the total number of the wells from each of which both the first signal and the second signal have been detected.

8. The detection method according to any one of claims 1 to 7, wherein a difference between a melting temperature Tm of the first specific binding substance and the first nucleic acid and a melting temperature Tm of the second specific binding substance and the second nucleic acid is less than or equal to 10 degrees Celsius.

9. A detection method comprising:

bringing liquid including a target nucleic acid into contact with a device including a well array having a plurality of wells, and introducing the target nucleic acid into a well among the plurality of wells in a manner to cause at most one molecule to be included per well;

sealing the well to prevent the target nucleic acid from moving between the plurality of wells;

amplifying, in the well, a signal derived from the target nucleic acid; and

detecting the signal emitted from the well, wherein

the target nucleic acid includes a first nucleic acid, a second nucleic acid that is complementary to the first nucleic acid, and a double-stranded nucleic acid resulting from complementary binding of the first nucleic acid and the second nucleic acid,

in the sealing, the double-stranded nucleic acid has a double-strand when sealed within the well, and

in the amplifying of the signal, a first signal is emitted as a result of a first specific binding substance being bound to the first nucleic acid.

**Patentansprüche**

1. Nachweisverfahren, umfassend:

   In-Kontakt-Bringen einer Flüssigkeit, die eine Zielnukleinsäure umfasst, mit einer Vorrichtung umfassend eine Anordnung von Vertiefungen, die eine Vielzahl von Vertiefungen aufweist, und

   Einführen der Zielnukleinsäure in eine Vertiefung aus der Vielzahl von Vertiefungen auf eine Weise, dass höchstens ein Molekül pro Vertiefung umfasst ist;

   Verschließen der Vertiefung, um zu verhindern, dass sich die Zielnukleinsäure zwischen der Vielzahl von Vertiefungen bewegt;

   Amplifizieren eines von der Zielnukleinsäure stammenden Signals in der Vertiefung; und

   Nachweisen des aus der Vertiefung emittierten Signals,

   wobei

   die Zielnukleinsäure eine erste Nukleinsäure, eine zweite Nukleinsäure, die komplementär zu der ersten Nukleinsäure ist, und eine doppelsträngige Nukleinsäure umfasst, die aus komplementären Binden der ersten Nukleinsäure und der zweiten Nukleinsäure resultiert,

   bei dem Verschließen die doppelsträngige Nukleinsäure einen Doppelstrang aufweist, wenn sie in der Vertiefung verschlossen wird,

   bei dem Amplifizieren des Signals ein erstes Signal als Ergebnis eines Bindens einer ersten spezifischen Bindungssubstanz an die erste Nukleinsäure abgegeben wird und ein zweites Signal als Ergebnis eines Bindens einer zweiten spezifischen Bindungssubstanz an die zweite Nukleinsäure abgegeben wird, und das erste Signal und das zweite Signal verschieden sind.

2. Nachweisverfahren nach Anspruch 1, wobei
   jedes des ersten Signals und des zweiten Signals ein Lumineszenzsignal ist, und eine Wellenlänge des ersten Signals und eine Wellenlänge des zweiten Signals verschieden sind.

3. Nachweisverfahren nach Anspruch 1 oder 2, wobei ein invasiver Spaltungsassay für das Amplifizieren des Signals verwendet wird.

4. Nachweisverfahren nach einem der Ansprüche 1 bis 3, wobei

   bei dem Nachweisen die Vertiefung, aus der nur das erste Signal nachgewiesen wurde, als nur die erste einzelsträngige Nukleinsäure umfassend bestimmt wird,

   die Vertiefung, aus der nur das zweite Signal nachgewiesen wurde, als nur die zweite einzelsträngige Nukleinsäure umfassend bestimmt wird und

   die Vertiefung, aus der sowohl das erste Signal als auch das zweite Signal nachgewiesen wurden, als die doppelsträngige Nukleinsäure umfassend bestimmt wird.

5. Nachweisverfahren nach einem der Ansprüche 1 bis 4, wobei das Nachweisen ferner Zählen einer Gesamtzahl von Vertiefungen, aus denen jeweils das Signal nachgewiesen wurde, unter der Vielzahl von Vertiefungen umfasst.

6. Nachweisverfahren nach Anspruch 5, wobei das Zählen Zählen einer Gesamtzahl von Vertiefungen, aus denen jeweils nur das erste Signal nachgewiesen wurde, einer Gesamtzahl von Vertiefungen, aus denen jeweils nur das zweite Signal nachgewiesen wurde, und einer Gesamtzahl von Vertiefungen, aus denen jeweils sowohl das erste Signal als auch das zweite Signal nachgewiesen wurden, unter der Vielzahl von Vertiefungen umfasst.

7. Nachweisverfahren nach Anspruch 6, wobei ein Verhältnis einer Gesamtzahl von doppelsträngigen Nukleinsäuren, von denen jede die doppelsträngige Nukleinsäure ist, zu einer Summe einer Gesamtzahl von ersten Nukleinsäuren, von denen jede die erste Nukleinsäure ist und einzelsträngig ist, und der Gesamtzahl der doppelsträngigen Nukleinsäuren in der Flüssigkeit, berechnet wird aus der Gesamtzahl der Vertiefungen, aus denen jeweils nur das erste Signal nachgewiesen wurde, und der Gesamtzahl der Vertiefungen, aus denen jeweils sowohl das erste Signal als auch das zweite Signal nachgewiesen wurden.

8. Nachweisverfahren nach einem der Ansprüche 1 bis 7, wobei eine Differenz zwischen einer Schmelztemperatur Tm der ersten spezifischen Bindungssubstanz und der ersten Nukleinsäure und einer Schmelztemperatur Tm der zweiten spezifischen Bindungssubstanz und der zweiten Nukleinsäure kleiner oder gleich 10 Grad Celsius ist.

9. Nachweisverfahren, umfassend:

In-Kontakt-Bringen einer Flüssigkeit, die eine Zielnukleinsäure umfasst, mit einer Vorrichtung umfassend eine Anordnung von Vertiefungen, die eine Vielzahl von Vertiefungen aufweist, und Einführen der Zielnukleinsäure in eine Vertiefung aus der Vielzahl von Vertiefungen auf eine Weise, dass höchstens ein Molekül pro Vertiefung umfasst ist;
Verschließen der Vertiefung, um zu verhindern, dass sich die Zielnukleinsäure zwischen der Vielzahl von Vertiefungen bewegt;
Amplifizieren eines von der Zielnukleinsäure stammenden Signals in der Vertiefung; und
Nachweisen des aus der Vertiefung emittierten Signals,
wobei
die Zielnukleinsäure eine erste Nukleinsäure, eine zweite Nukleinsäure, die komplementär zu der ersten Nukleinsäure ist, und eine doppelsträngige Nukleinsäure umfasst, die aus komplementären Binden der ersten Nukleinsäure und der zweiten Nukleinsäure resultiert,
bei dem Verschließen die doppelsträngige Nukleinsäure einen Doppelstrang aufweist, wenn sie in der Vertiefung verschlossen wird,
bei dem Amplifizieren des Signals ein erstes Signal als Ergebnis eines Bindens einer ersten spezifischen Bindungssubstanz an die erste Nukleinsäure abgegeben wird.

**Revendications**

1. Procédé de détection comprenant :

la mise en contact d'un liquide comprenant un acide nucléique cible avec un dispositif comprenant un réseau de puits ayant une pluralité de puits, et
l'introduction de l'acide nucléique cible dans un puits parmi la pluralité de puits de manière à provoquer l'inclusion d'au plus une molécule par puits ;
le scellement du puits pour empêcher l'acide nucléique cible de se déplacer entre la pluralité de puits ;
l'amplification, dans le puits, d'un signal dérivé de l'acide nucléique cible ; et
la détection du signal émis par le puits,
dans lequel
l'acide nucléique cible comprend un premier acide nucléique, un second acide nucléique qui est complémentaire du premier acide nucléique, et un acide nucléique double brin résultant de la liaison complémentaire du premier acide nucléique et du second acide nucléique,
lors du scellement, l'acide nucléique double brin présente un double brin lorsqu'il est scellé dans le puits,
lors de l'amplification du signal, un premier signal est émis suite à la liaison d'une première substance à liaison spécifique au premier acide nucléique, et un second signal est émis suite à la liaison d'une seconde substance à liaison spécifique au second acide nucléique, et
le premier signal et le second signal sont différents.

2. Procédé de détection selon la revendication 1, dans lequel
chacun parmi le premier signal et le second signal est un signal de luminescence, et une longueur d'onde du premier signal et une longueur d'onde du second signal sont différentes.

**3.** Procédé de détection selon la revendication 1 ou 2, dans lequel un essai de clivage invasif est utilisé pour l'amplification du signal.

**4.** Procédé de détection selon l'une quelconque des revendications 1 à 3, dans lequel lors de la détection, il est déterminé que le puits à partir duquel seul le premier signal a été détecté ne comprend que le premier acide nucléique qui est simple brin,

il est déterminé que le puits à partir duquel seul le second signal a été détecté ne comprend que le second acide nucléique qui est simple brin, et

il est déterminé que le puits à partir duquel à la fois le premier signal et le second signal ont été détectés comprend l'acide nucléique double brin.

**5.** Procédé de détection selon l'une quelconque des revendications 1 à 4, dans lequel la détection comprend en outre le comptage, parmi la pluralité de puits, d'un nombre total de puits à partir de chacun desquels le signal a été détecté.

**6.** Procédé de détection selon la revendication 5, dans lequel le comptage comprend le comptage, parmi la pluralité de puits, d'un nombre total de puits à partir de chacun desquels seul le premier signal a été détecté, d'un nombre total de puits à partir de chacun desquels seul le second signal a été détecté, et d'un nombre total de puits à partir de chacun desquels à la fois le premier signal et le second signal ont été détectés.

**7.** Procédé de détection selon la revendication 6, dans lequel un rapport d'un nombre total d'acides nucléiques double brin dont chacun est l'acide nucléique double brin par rapport à une somme d'un nombre total de premiers acides nucléiques dont chacun est le premier acide nucléique et est simple brin et le nombre total des acides nucléiques double brin dans le liquide est calculé à partir du nombre total des puits à partir de chacun desquels seul le premier signal a été détecté et du nombre total des puits à partir de chacun desquels à la fois le premier signal et le second signal ont été détectés.

**8.** Procédé de détection selon l'une quelconque des revendications 1 à 7, dans lequel une différence entre une température de fusion Tm de la première substance à liaison spécifique et du premier acide nucléique et une température de fusion Tm de la seconde substance à liaison spécifique et du second acide nucléique est inférieure ou égale à 10 degrés Celsius.

**9.** Procédé de détection comprenant :

la mise en contact d'un liquide comprenant un acide nucléique cible avec un dispositif comprenant un réseau de puits ayant une pluralité de puits, et l'introduction de l'acide nucléique cible dans un puits parmi la pluralité de puits de manière à provoquer l'inclusion d'au plus une molécule par puits ;
le scellement du puits pour empêcher l'acide nucléique cible de se déplacer entre la pluralité de puits ;
l'amplification, dans le puits, d'un signal dérivé de l'acide nucléique cible ; et
la détection du signal émis par le puits, dans lequel
l'acide nucléique cible comprend un premier acide nucléique, un second acide nucléique qui est complémentaire du premier acide nucléique, et un acide nucléique double brin résultant de la liaison complémentaire du premier acide nucléique et du second acide nucléique,
lors du scellement, l'acide nucléique double brin présente un double brin lorsqu'il est scellé dans le puits, et
lors de l'amplification du signal, un premier signal est émis suite à la liaison d'une première substance à liaison spécifique au premier acide nucléique.

# FIG.1

# FIG.2

**3**

**4**

# FIG.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2019222153 A
- WO 2015115635 A1
- US 2014274786 A1
- WO 2009054474 A1

### Non-patent literature cited in the description

- **OLMEDILLAS-LOPEZ S. et al.** Current and Emerging Applications of Droplet Digital PCR. *Oncology. Mol Diagn Ther.,* October 2017, vol. 21 (5), 493-510